# EUROPÄISCHE PATENTSCHRIFT

(11) **EP 2 560 961 B1**
(45) Veröffentlichungstag und Bekanntmachung des Hinweises auf die Patenterteilung: **16.11.2016**
(21) Anmeldenummer: 11714986.4
(22) Anmeldetag: 18.04.2011
(51) Int. Cl.: C07D 231/16

(54) **VERFAHREN ZUM HERSTELLEN VON 5-FLUOR-1-ALKYL-3-FLUORALKYL-1H-PYRAZOL-4-CARBONSÄURECHLORIDEN UND -FLUORIDEN**
METHOD FOR PRODUCING 5-FLUOR-1-ALKYL-3-FLUOROALKYL-1H-PYRAZOL-4-CARBOXYLIC ACID AND CHLORIDES AND FLUORIDES
PROCEDE DE DE FABRICATION DE CHLORURES ET DE FLUORURES D' ACIDE 5-FLUORO-1-ALKYL-3-FLUOROALKYL-1H-PYRAZOL-4-CARBOXYLIQUE

(30) Priorität: 23.04.2010 US 327269 P; 23.04.2010 EP 10160885
(43) Veröffentlichungstag der Anmeldung: 27.02.2013
(73) Patentinhaber: Bayer Intellectual Property GmbH, 40789 Monheim (DE)
(72) Erfinder: PAZENOK, Sergii, 42699 Solingen (DE); LUI, Norbert, 51519 Odenthal (DE); GARY, Stephanie, F-69410 Champagne au Mont D´OR (FR)
(74) Vertreter: BIP Patents
(86) Internationale Anmeldenummer: PCT/EP2011/056119
(87) Internationale Veröffentlichungsnummer: WO 2011/131615

(56) Entgegenhaltungen:
- WO-A1-2007/031212
- US-A1- 2006 089 399

## Beschreibung

Die vorliegende Erfindung betrifft ein neues Verfahren zum Herstellen von 5-Fluor-1-alkyl-3-fluoralkyl-1H-pyrazol-4-carbonsäurehalogeniden und die in diesem Verfahren auftretenden Zwischenstufen, Ethyl 5-Chlor-1-methyl-3-difluormethyl-1H-pyrazol-4-carboxylat und Ethyl 5-Fluor-1-methyl-3-difluormethyl-1 H-pyrazol-4-carboxylat.

5-Fluor-1-alkyl-3-fluoralkyl-1H-pyrazol-4-carbonsäurehalogenide sind wichtige Zwischenstufen in der Synthese von Pflanzenschutzmitteln (vgl. z.B. bislang unveröffentlichte Europäische Patentanmeldung EP-A 2 251 331 und WO-A 2007/087906).

Pyrazolcarbonsäurechloride stellt man üblicherweise, wie bereits in WO-A 1992/012970 beschrieben, durch Umsetzung von Carbonsäuren mit einem Chlorierungsmittel dar. Ein Vorteil dieses Verfahrens beruht darauf, dass die zugrundeliegenden Carbonsäuren einfach zugänglich und damit im technischen Maßstab verfügbar sind. Diese Voraussetzung ist bei der Herstellung der substituierten Pyrazolcarbonsäurechloride nicht gegeben, da die entsprechenden substituierten Carbonsäuren nicht leicht zugänglich sind. Ein anderes Verfahren, welches in der bislang unveröffentlichten Europäischen Patentanmeldung 09176426 beschrieben ist, gelangt zu den Säurechloriden ausgehend von Pyrazolylcarbaldehyden über eine mehrstufige Synthese.

Verfahren zum Austausch von Chlor gegen Fluor (Halexprozesse) sind insbesondere für 5-Chloro-1,3-dilakyl-1H-pyrazol-4-carbosäurechloride bekannt (vgl. z.B. WO 2007/031212 und EP-A 0 776 889). Hierbei wird das Säurechlorid in das Säurefluorid umgewandelt und dadurch zusätzlich die Fluorierung beschleunigt. Falls die Aktivierungsgruppe jedoch der Aldehyd (CHO) anstelle des Säurechlorids (COCl) oder des Säurefluorids (COF) ist, liefert die Umsetzung mit Kaliumfluorid nur sehr niedrige Ausbeuten. Zum Beispiele wird 5-Fluor-1,3-dimethyl-1H-pyrazol-4-carbaldehyd durch Umsetzung von 1,3-Dimethyl-5-chlorpyrazol-4-carbaldehyd mit Kaliumfluorid nur in 24 % Ausbeute (EP-A 0 776 889) erhalten. Als Ursache für die schlechte Ausbeute kommt auch die niedrige thermische Stabilität von Pyrazolaldehyden infrage (vgl. EP-A 1 364 946).

Der vorliegenden Erfindung liegt somit die Aufgabe zugrunde, einen Weg zur Herstellung von 5-Fluor-1-alkyl-3-fluoralkyl-1H-pyrazol-4-carbonsäurehalogeniden zur Verfügung zu stellen, der die im Stand der Technik beschriebenen Nachteile nicht aufweist.

Die erfindungsgemäße Aufgabe wurde nun gelöst, durch ein Verfahren zum Herstellen von 5-Fluor-1-alkyl-3-fluoralkyl-1H-pyrazol-4-carbonsäurehalogeniden der Formel (I) 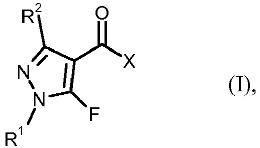 in welcher R¹ für C₁-C₆-Alkyl, R² für C₁-C₅-Fluoralkyl und X für Fluor oder Chlor steht, umfassend die Schritte
(1) Chlorierung von Alkyl 1-alkyl-3-fluoralkyl-1H-pyrazol-4-carboxylaten der Formel (II), 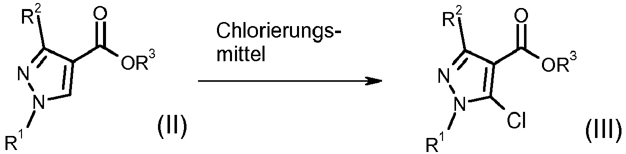 in der R¹, R² die oben angegebenen Bedeutungen haben und R³ eine lineare oder verzweigte C₁₋₁₂-Alkylgruppe ist, in Gegenwart eines Chlorierungsmittels zu Alkyl 5-Chlor-1-alkyl-3-fluoralkyl-1H-pyrazol-4-carboxylaten der Formel (III), in der R¹, R² und R³ die oben angegebenen Bedeutungen haben;
(2a-i) Fluorierung der Alkyl 5-Chlor-1-alkyl-3-fluoralkyl-1H-pyrazol-4-carboxylate der Formel (III) in Gegenwart eines Fluorierungsmittels der Formel (IV), wobei M⁺ für Li⁺, Na⁺, K⁺, Cs⁺, Alk₄N⁺ steht und Alk für C₁-C₄-Alkyl steht, zu Alkyl 5-Fluor-1-alkyl-3-fluoralkyl-1H-pyrazol-4-carboxylaten der Formel (V) und
(2a-ii) Hydrolyse der Alkyl 5-Fluor-1-alkyl-3-fluoralkyl-1H-pyrazol-4-carboxylate der Formel (V) zu 5-Fluor-1-alkyl-3-fluoralkyl-1H-pyrazol-4-carbonsäuren der Formel (VIa) und
(2a-iii) anschließende Halogenierung zu 5-Fluor-1-alkyl-3-fluoralkyl-1H-pyrazol-4-carbonsäurehalogeniden der Formel (I), wobei X für Fluor oder Chlor steht; 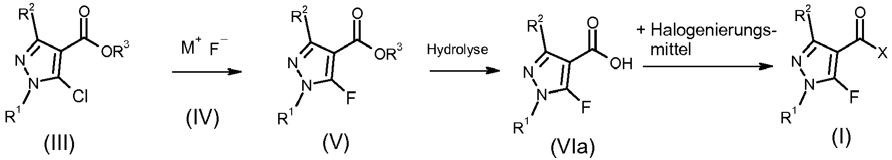 oder
(2b-i) Hydrolyse der Alkyl 5-Chlor-1-alkyl-3-fluoralkyl-1H-pyrazol-4-carboxylate der Formel (III) zu 5-Chlor-1-alkyl-3-fluoralkyl-1H-pyrazol-4-carbonsäuren der Formel (VI b),
(2b-ii) anschließende Halogenierung zu 5-Chlor-1-alkyl-3-fluoralkyl-1H-pyrazol-4-carbonsäure-halogeniden der Formel (VII) und 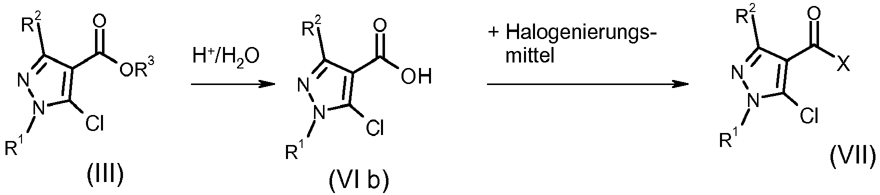
(2b-iii) Fluorierung der 5-Chlor-1-alkyl-3-fluoralkyl-1H-pyrazol-4-carbonsäurehalogenide der Formel (VII) in Gegenwart eines Fluorierungsmittels der Formel (IV), wobei M⁺ für Li⁺, Na⁺, K⁺, Cs⁺, Alk₄N⁺ steht und Alk für C₁-C₄-Alkyl steht zu den 5-Fluor-1-alkyl-3-fluoralkyl-1H-pyrazol-4-carbonsäurefluoriden der Formel (I), wobei X für Fluor steht.

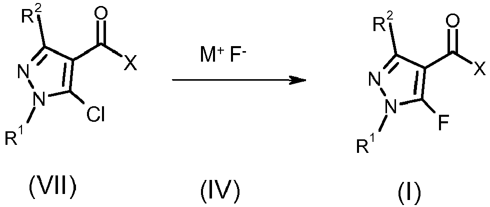

Es war nicht vorauszusehen, ob die Fluorierung von Alkyl 5-Chlor-1-alkyl-3-fluoralkyl-1H-pyrazol-4-carboxylaten mit Metallfluoriden erfolgreich sein könnte. Daher ist es als überraschend anzusehen, dass die Fluorierung von 5-Chlor-1-alkyl-3-fluoralkyl-1H-pyrazol-4-carboxylaten mit Metallfluoriden selektiv und in hoher Ausbeute zu neuen Alkyl 5-Fluor-1-alkyl-3-fluoralkyl-1H-pyrazol-4-carboxylaten führt.

Das erfindungsgemäße Verfahren kann durch das folgende Schema (I) veranschaulicht werden:

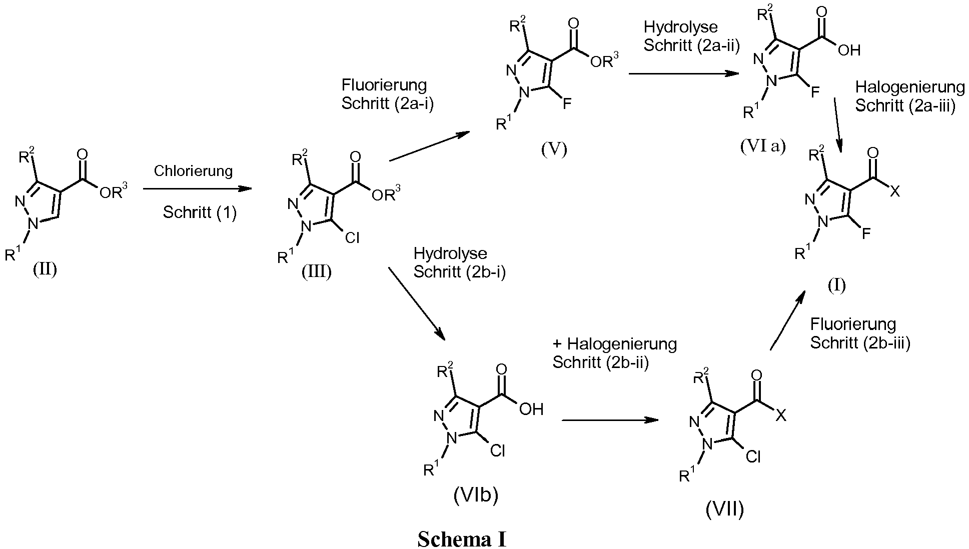

Die bei der Durchführung des erfindungsgemäßen Verfahrens als Ausgangsstoffe verwendeten Alkyl 1-alkyl-3-fluoralkyl-1H-pyrazol-4-carboxylate sind durch die Formel (II) allgemein definiert. Die Reste R¹ und R³ stehen in dieser Formel (II) bevorzugt für Methyl, Ethyl, n-Propyl, Isopropyl, Butyl, Pentyl, besonders bevorzugt für Methyl, Ethyl, n-Propyl. Der Rest R² steht für C₁-C₅-Fluoralkyl, welches mit mindestens einem Fluoratom bis hin zur Perfluorierung substituiert ist. Wenn das Fluoralkyl nicht perfluoriert ist, können weitere Halogenatome wie Chlor und Brom, bevorzugt Chlor, als weitere Substituenten vorhanden sein. R² steht bevorzugt für CF₂H, CF₃, CF₂Cl, CCl₂F, C₂F₅, C₃F₇, besonders bevorzugt für CF₂H und CF₃. Ganz besonders bevorzugt setzt man Ethyl 1-methyl-3-difluormethyl-1H-pyrazol-4-carboxylate (II-1) als Ausgangsstoff ein. Die Gruppe X ist ein Halogenatom, welches ausgewählt ist aus Fluor und Chlor.

Erfolgt die Herstellung der 5-Fluor-1-alkyl-3-fluoralkyl-1H-pyrazol-4-carbonsäurehalogeniden der Formel (I) aus den 5-Fluor-1-alkyl-3-fluoralkyl-1H-pyrazol-4-carbonsäuren der Formel (VIa), so kann durch den abschließenden Halogenierungsschritt (2a-iii), je nach Wahl des Halogenierungsreagenzes, entweder das entsprechende Chlorid (X=Cl) als auch das Fluorid (X=F) erhalten werden.

Bei der alternativen erfindungsgemäßen Herstellung der 5-Fluor-1-alkyl-3-fluoralkyl-1H-pyrazol-4-carbonsäurehalogenide der Formel (I) durch Fluorierung der 5-Chlor-1-alkyl-3-fluoralkyl-1H-pyrazol-4-carbonsäurehalogenide der Formel (VII) in Gegenwart eines Fluorierungsmittels der Formel (IV), werden nur 5-Fluor-1-alkyl-3-fluoralkyl-1H-pyrazol-4-carbonsäurefluoride der Formel (I) mit X=F erhalten. Diese können jedoch anschließend durch geeignete Halogenaustauschreaktionen, zum Beispiel mit SiCl₄, zu den entsprechenden Chloriden (X=Cl) umgewandelt werden.

Alkyl 1-alkyl-3-polyfluoralkyl-1H-pyrazol-4-carboxylate der Formel (II) sind bekannt oder lassen sich nach bekannten Verfahren herstellen (WO-A 2005/23690, WO-A 2008/022777).

### Schritt 1 Chlorierung

Die Chlorierung von Alkyl 1-alkyl-3-fluoralkyl-1H-pyrazol-4-carboxylaten der Formel (II) zu Alkyl 5-Chlor-1-alkyl-3-fluoralkyl-1H-pyrazol-4-carboxylaten der Formel (III) erfolgt erfindungsgemäß in Gegenwart eines Chlorierungsmittels. Geeignete Chlorierungsmittel, ohne Anspruch auf Vollständigkeit zu erheben, sind beispielsweise Cl₂, SO₂Cl₂, SOCl₂, N-Chlorsuccinimid oder ein Gemisch derselben. Bevorzugt werden Cl₂, SO₂Cl₂, oder ein Gemisch derselben als Chlorierungsmittel eingesetzt. Besonders bevorzugt ist SO₂Cl₂. Das Alkyl 1-alkyl-3-fluoralkyl-1H-pyrazol-4-carboxylate der Formel (II) und das Chlorierungsmittel werden in einem Molverhältnis von 1 : 10 bis 1 :1, bevorzugt 1 : 5 bis 1 : 2 eingesetzt.

Die Chlorierung erfolgt üblicherweise bei Temperaturen von -10 bis 150°C, vorzugsweise von 20 bis 100°C und kann bei Normaldruck oder unter Überdruck durchgeführt werden.

Erfolgt die Chlorierung in der Gasphase, so kann sie gegebenenfalls in Gegenwart eines inerten Verdünnungsgases wie z.B. Stickstoff, Kohlendioxyd oder Edelgase durchgeführt werden.

Die Chlorierung kann in Substanz oder in Gegenwart eines Verdünnungsmittels erfolgen, das sich unter den vorherrschenden Reaktionsbedingungen inert verhält. Man kann als Verdünnungsmittel beispielsweise einfach oder mehrfach chlorierte, aliphatische oder aromatische Kohlenwasserstoffe oder Gemische derselben einsetzen. Beispiele für geeignete Verdünnungsmittel sind Chlorbenzol, Dichlorbenzole, Trichlorbenzole, Chlortoluole, Chlorbenzotrifluoride, Methylenchlorid, Dichlorethan, Chloroform, Tetrachlorkohlenstoff. Bevorzugte Verdünnungsmittel sind Chlorbenzol, 1,2-Dichlorbenzol, 1,3-Dichlorbenzol, 1,4-Dichlorbenzol, 1,2,3-Trichlorbenzol, 1,2,4-Trichlorbenzol, 4-Chlor-trifluormethylbenzol, 1,3,5-Trichlorbenzol, 2-Chlortoluol, 3-Chlortoluol, 4-Chlortoluol oder ein Gemisch derselben. Besonders bevorzugt verwendet man Chlorbenzol.

### Schritte 2(a-iii) und 2(b-ii): Bildung des Säurehalogenids

Die Umsetzung von 5-Chlor-1-Alkyl-3-fluoralkyl-1H-pyrazol-4-carbonsäuren der Formel (VI b) zum den 5-Chlor-1-alkyl-3-fluoralkyl-1H-pyrazol-4-carbonsäurechloriden der Formel (VII) oder 5-Fluor-1-Alkyl-3-fluoralkyl-1H-pyrazol-4-carbonsäuren der Formel (VI a) zum 5-Fluor-1-alkyl-3-fluoralkyl-1H-pyrazol-4-carbonsäurechloriden der Formel (I), wobei X=Cl, erfolgt erfindungsgemäß in Gegenwart eines Chlorierungsmittels. Geeignete Chlorierungsmittel, ohne Anspruch auf Vollständigkeit zu erheben, sind beispielsweise SOCl₂, COCl₂, Diphosgen, Triphosgen, POCl₃, ClCO-COCl oder ein Gemisch derselben. Bevorzugt werden SOCl₂, COCl₂, ClCO-COCl oder ein Gemisch derselben als Chlorierungsmittel eingesetzt. Besonders bevorzugt sind SOCl₂. und COCl₂. Die 5-Chlor-1-alkyl-3-fluoralkyl-1H-pyrazol-4-carbonsäuren der Formel (VI b) oder die 5-Fluor-1-alkyl-3-fluoralkyl-1H-pyrazol-4-carbonsäuren der Formel (VIa) und das Chlorierungsmittel werden in einem Molverhältnis von 1 : 5 bis 1 :1, bevorzugt 1 : 2 bis 1 : 1,05 eingesetzt.

Die Chlorierung kann in Substanz oder in Gegenwart eines Verdünnungsmittels erfolgen, das sich unter den vorherrschenden Reaktionsbedingungen inert verhält. Man kann als Verdünnungsmittel beispielsweise einfach oder mehrfach chlorierte aliphatische oder aromatische Kohlenwasserstoffe oder Gemische derselben einsetzen. Beispiele für geeignete Verdünnungsmittel sind Chlorbenzol, Dichlorbenzole, Trichlorbenzole, Chlortoluole, Chlorbenzotrifluoride, Methylenchlorid, Dichlorethan, Chloroform, Tetrachlorkohlenstoff. Bevorzugte Verdünnungsmittel sind Chlorbenzol, 1,2-Dichlorbenzol, 1,3-Dichlorbenzol, 1,4-Dichlorbenzol, 1,2,3-Trichlorbenzol, 1,2,4-Trichlorbenzol, 4-Chlor-trifluormethylbenzol, 1,3,5-Trichlorbenzol, 2-Chlortoluol, 3-Chlortoluol, 4-Chlortoluol oder ein Gemisch derselben. Besonders bevorzugt verwendet man Chlorbenzol.

Die Umsetzung von 5-Chlor-1-Alkyl-3-fluoralkyl-1H-pyrazol-4-carbonsäuren der Formel (VI b) zum den 5-Chlor-1-alkyl-3-fluoralkyl-1H-pyrazol-4-carbonsäurefluoriden der Formel (VII) oder 5-Fluor-1-Alkyl-3-fluoralkyl-1H-pyrazol-4-carbonsäuren der Formel (VI a) zum 5-Fluor-1-alkyl-3-fluoralkyl-1H-pyrazol-4-carbonsäurefluoriden der Formel (I), wobei X=F steht, erfolgt erfindungsgemäß in Gegenwart eines Fluorierungsmittels. Geeignete Florierungsmittel, ohne Anspruch auf Vollständigkeit zu erheben, sind beispielsweise SF₄, DAST, Deoxofluor, TFEDMA (HCF₂-CF₂NMe₂), Difluorphosgen. Bevorzugt werden DAST oder TFEDMA (HCF₂CF₂NMe2 als Fluorierungsmittel eingesetzt. Besonders bevorzugt ist TFEDMA. Die 5-Chlor-1-alkyl-3-fluoralkyl-1H-pyrazol-4-carbonsäuren der Formel (VIb) oder die 5-Fluor-1-alkyl-3-fluoralkyl-1H-pyrazol-4-carbonsäuren der Formel (VIa) und das Fluorierungsmittel werden in einem Molverhältnis von 1 : 2 bis 1 :1, bevorzugt 1 : 2 bis 1 : 1,5 eingesetzt.

Die Fluorierung kann in Substanz oder in Gegenwart eines Verdünnungsmittels erfolgen, das sich unter den vorherrschenden Reaktionsbedingungen inert verhält. Man kann als Verdünnungsmittel beispielsweise einfach oder mehrfach chlorierte aliphatische oder aromatische Kohlenwasserstoffe oder Gemische derselben einsetzen. Beispiele für geeignete Verdünnungsmittel sind Chlorbenzol, Dichlorbenzole, Trichlorbenzole, Chlortoluole, Chlorbenzotrifluoride, Methylenchlorid, Dichlorethan, Chloroform, Tetrachlorkohlenstoff. Bevorzugte Verdünnungsmittel sind Chlorbenzol, 1,2-Dichlorbenzol, 1,3-Dichlorbenzol, Dichlorethan, Dichlormethan oder ein Gemisch derselben. Besonders bevorzugt verwendet man Chlorbenzol und Dichlormethan.

### Schritte (2a-i) oder (2b-iii): Fluorierung

Die Fluorierung der Alkyl 5-Chlor-1-alkyl-3-fluoralkyl-1H-pyrazol-4-carboxylate der Formel (III) zu Alkyl 5-Fluor-1-alkyl-3-fluoralkyl-1H-pyrazol-4-carboxylaten der Formel (V) gemäß Schritt (2a-i) erfolgt erfindungsgemäß in Gegenwart eines Fluorierungsmittels der Formel (IV).

In einer erfindungsgemäßen alternativen Ausführungsform des Verfahrens, welches im obigen Schema I als Schritt (2b-iii) dargestellt ist, werden die 5-Chlor-1-alkyl-3-fluoralkyl-1H-pyrazol-4-carbonsäurehalogenide der Formel (VII) abschließend in Gegenwart eines Fluorierungsmittels der Formel (IV) zu den 5-Fluor-1-alkyl-3-fluoralkyl-1H-pyrazol-4-carbonsäurehalogeniden der Formel (I) umgesetzt.

M⁺ F⁻ (IV)

In Formel (IV) steht M⁺ für ein Alkalimetall- oder Ammoniumkation, vorzugsweise für Li⁺, Na⁺, K⁺, Cs⁺, Alk₄N⁺, oder ein Gemisch derselben , wobei Alk für C₁-C₄-Alkyl steht. Besonders bevorzugt werden Natrium- und Kaliumfluorid oder ein Gemisch derselben als Fluorierungsmittel eingesetzt.

Natrium- und Kaliumfluorid, sind bekannte Synthesechemikalien und kommerziell zugänglich.

Die Reaktionstemperaturen können bei der Durchführung des erfindungsgemäßen Fluorierungsschritts in einem größeren Bereich variiert werden. Im Allgemeinen arbeitet man bei Temperaturen von 120°C bis 200°C, bevorzugt bei Temperaturen von 110°C bis 180°C.

Die Reaktionszeit kann in Abhängigkeit von der Reaktivität der Edukte bis zu 10 Stunden betragen, wobei der Abbruch der Reaktion bei vollständigem Umsatz auch schon früher erfolgen kann. Bevorzugt sind Reaktionszeiten von 3-5 Stunden.

Bei der Durchführung des erfindungsgemäßen Verfahrens setzt man pro mol ein austauschbares Chloratom in Alkyl 5-Chlor-1-alkyl-3-fluoralkyl-1H-pyrazol-4-carboxylate der Formel (III) oder in 5-Chlor-1-alkyl-3-fluoralkyl-1H-pyrazol-4-carbonsäurehalogenid der Formel (VII) im Allgemeinen zwischen 0,8 und 1,8 mol, bevorzugt zwischen 1 und 1,5 mol, an Fluorierungsmittel der Formel (IV) ein.

Die Reaktion kann in Substanz oder in Gegenwart eines Lösungsmittels durchgeführt werden. Geeignete Lösungsmittel sind: Sulfolan, Dimethylsulphoxid (DMSO), Dimethylacetamid, Dimethylformamid (DMF), N-Methylpyrrolidon (NMP), 1,3-Dimethylimidazolinon, Dichlormethan, Chloroform, Dichlorethan oder Trichlorethan; Ketone, wie Aceton, Butanon, Methyl-isobutylketon oder Cyclohexanon; Nitrile, wie Acetonitril, Propionitril, n- oder i-Butyronitril oder Hexamethylphosphorsäuretriamid. Besonders bevorzugt verwendet man Sulfolan, DMSO, Dimethylacetamid, DMF oder NMP.

Die Fluorierung kann durch Zusatz von Phasen-Transfer-Katalysatoren beschleunigt werden.

Zur Durchführung des erfindungsgemäßen Verfahrens eignen sich insbesondere quarternäre Ammonium-, Phosphoniumverbindungen oder Amidophosphonium-Salze als Phasen-Transfer-Katalysatoren. Beispielhaft seinen Verbindungen wie Tetramethylammonimchlorid oder Bromid, Tetrabutylammoniumchlorid, Trimethylbenzylammoniumchlorid, Tetrabutylammoniumbromid, Tetrabutylphosphoniumchlorid, Tetrabutylphosphoniumbromid, Tetraphenylphosphoniumchlorid oder Tetraphenylphosphoniumbromid, Tetrakis(dimethylamino)phosphoniumchlorid oder -bromid, Tetrakis(diethylamino)phosphoniumchlorid oder -bromid, Tris(diethylamino)(dimethylamino)-phosphoniumchlorid oder -bromid, Tris(dimethylamino)(dihexylamino)phosphoniumchlorid oder - bromid, Tris(diethylamino)(dihexylamino)phosphoniumchlorid oder -bromid, Hexaalkyguanidiniusalze oder Polyethylenglykoldimethylethern der Kettenlängen r von 6 bis 17 und einer mittleren Molmasse von 500 g/mol, genannt.

Bei der erfindungsgemäßen alternativen Ausführungsform des Verfahrens, welches im obigen Schema I als Schritt (2b-iii) dargestellt ist, werden die 5-Chlor-1-alkyl-3-fluoralkyl-1H-pyrazol-4-carbonsäurehalogenide der Formel (VII) abschließend in Gegenwart eines Fluorierungsmittels der Formel (IV) zu den 5-Fluor-1-alkyl-3-fluoralkyl-1H-pyrazol-4-carbonsäurehalogeniden der Formel (I) umgesetzt. In diesen Fall ist der Halogenierungsschritt (2b-ii) vorzugsweise eine Chlorierung (X=Cl) und die daraus erhaltenen 5-Chlor-1-alkyl-3-fluoralkyl-1H-pyrazol-4-carbonsäurechloride der Formel (VII) werden in Gegenwart eines Fluorierungsmittels der Formel (IV) zu den 5-Fluor-1-alkyl-3-fluoralkyl-1H-pyrazol-4-carbonsäurefluoriden der Formel (I) mit X=F umgewandelt.

Sowohl die Alkyl 5-Fluor-1-alkyl-3-fluoralkyl-1H-pyrazol-4-carboxylate der Formel (V) als auch die 5-Fluor-1-alkyl-3-fluoralkyl-1H-pyrazol-4-carbonsäuren der Formel (VIa) sind wichtige Zwischenstufen in der Synthese von Pflanzenschutzmitteln (vgl. z.B. Europäische Patentanmeldung EP-A 2251331und WO-A 2007/087906).

### Schritt 2(b-i) und 2(a-ii) : Hydrolyse

Die Hydrolyse der Alkyl 5-Chlor-1-alkyl-3-fluoralkyl-1H-pyrazol-4-carboxylate der Formel (III) oder 5-Fluor-1-alkyl-3-fluoralkyl-1H-pyrazol-4-carboxylate der Formel (V) und die Herstellung 5-Chlor-1-alkyl-3-fluoralkyl-1H-pyrazol-4-carbonsäuren der Formel (VI b) oder 5-Chlor-1-alkyl-3-fluoralkyl-1H-pyrazol-4-carbonsäuren der Formel (VIa) und (VI b) und die anschließende Chlorierung zu den 5-Chlor-1-alkyl-3-fluoralkyl-1H-pyrazol-4-carbonsäurechloriden der Formel (VII), wobei X=Cl ist, oder zu den 5-Fluor-1-alkyl-3-fluoralkyl-1H-pyrazol-4-carbonsäurechloriden der Formel (I) wird, wie in WO-A 2005/123690 beschrieben, durchgeführt.

In einer bevorzugten Ausführungsform der vorliegenden Erfindung können die zuvor beschriebenen Schritte ohne zwischenzeitliche Isolierung der entstehenden Zwischenstufen durchlaufen werden.

Ein weiterer Gegenstand der vorliegenden Erfindung betrifft die bei dem Verfahren entstehenden Zwischenstufen Ethyl 5-Chlor-1-methyl-3-difluormethyl-1H-pyrazol-4-carboxylat (III-1) und Ethyl 5-Fluor-1-methyl-3-difluormethyl-1H-pyrazol-4-carboxylat (V-1), die bei der Umsetzung von Ethyl 1-Methyl-3-difluormethyl-1H-pyrazol-4-carboxylat nach dem erfindungsgemäßen Verfahren durchlaufen werden.

Das erfindungsgemäße Herstellen von 5-Fluor-1,3-dialkyl-1H-pyrazol-4-carbonsäurefluoriden der Formel (I) wird in den nachstehenden Beispielen beschrieben, welche die obige Beschreibung weiter illustrieren. Die Beispiele sind jedoch nicht in einschränkender Weise zu interpretieren.

### Herstellungsbeispiele

### Beispiel 1: Ethyl 5-chlor-1-methyl-3-difluormethyl-1H-pyrazol-4-carboxylate (III-1)

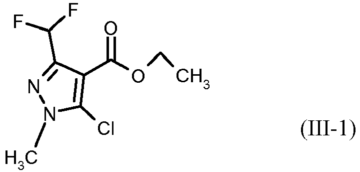

Unter Argon werden 20.25 g (100 mmol) Ethyl 1-methyl-3-difluormethyl-1H-pyrazol-4-carboxylat und 40 ml SO₂Cl₂ vorgelegt, das Gemisch wird auf 60°C° erhitzt und bei dieser Temperatur für 8 Stunden gerührt. Nach dem Verdünnen des Gemischs mit Ethylacetat , Waschen mit Wasser und Entfernen des Lösungsmittels im Vakuum erhält man 21 g des Produktes mit einer HPLC Reinheit von 94 % und einem Schmelzpunkt von 37-39°C.
¹H-NMR (CDCl₃): δ = 7.1 (1H, t), 4,3 (qw, 2H), 3,9 (s, 3H), 1,4 (t, 3H) ppm.
¹⁹F-NMR (CD₃CN): δ = -114,9 (2F, t) ppm.

### Beispiel 2: Ethyl 5-Fluor-1-methyl-3-difluormethyl-1H-pyrazol-4-carboxylat

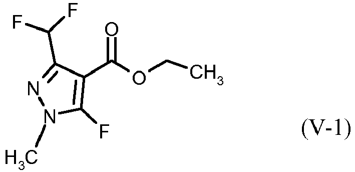

9,63 g (166 mmol) sprühgetrocknetes Kaliumfluorid, 23,8 g (100 mmol) Ethyl 5-Chlor-1-methyl-3-difluormethyl-1H-pyrazol-4-carboxylate (II-1) und 150 ml Sulpholan werden unter schnellem Rühren auf 190°C erhitzt. Die Reaktion ist nach 8 Stunden (GC-Kontrolle) abgeschlossen. Man kühlt auf Raumtemperatur ab, gibt Wasser bis zum einem Gesamtvolumen von 500 ml zu und wäscht das Reaktionsgemisch zweimal mit je 150 ml Ethylacetat. Die vereinigten, organischen Phasen werden mit gesättigter Natriumchloridlösung gewaschen, über Natriumsulfat getrocknet und eingeengt. Man erhält 20 g des Produktes mit einer Reinheit von 93 %. Ethyl 5-Fluor-1-methyl-3-difluormethyl-1H-pyrazol-4-carboxylate ist in der Form eines braunen Feststoffes, welcher ohne weitere Aufreinigung weiter umgesetzt wird.

### Beispiel 3: 5-Fluor-1-methyl-3-difluormethyl-1H-pyrazol-4-carbonsäurechlorid (I-1)

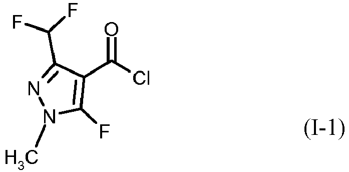

Eine Lösung von 22,3 g (100 mmol) Ethyl 5-Fluor-1-methyl-3-difluormethyl-1H-pyrazol-4-carboxylate in 100 ml Toluol und 50 ml 10 % NaOH werden 3 Std. bei Raumtemperatur gerührt. Die wässrige Phase wird abgetrennt und mit HCl auf pH 5 eingestellt. Nach dem Extrahieren des Produkts mit Chlorbenzol und azeotroper Trocknung der organische Phase werden zu der Lösung 23,8 g (200 mmol) SOCl₂ zugegeben und das Gemisch 2 Std. bei 80°C erhitzt. Nach dem Einengen des Reaktionsgemischs erhält man 20.1 g des Produkts als Öl mit der Reinheit (GC) von 98 %.
¹H-NMR (CD₃CN): δ = 6,88 (1H, t), 3,7 (3H, s) ppm.

### Beispiel 4: 5-Fluor-1-methyl-3-difluormethyl-1H-pyrazol-4-carbonsäurefluorid

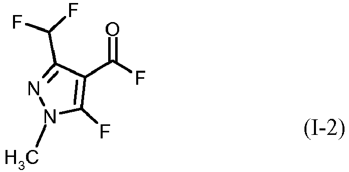

14,2 g (250 mmol) sprühgetrocknetes Kaliumfluorid, 22,8 g (100 mmol) 5-Chlor-1-methyl-3-difluormethyl-1H-pyrazol-4-carbonsäurechlorid und 40 ml Sulpholan werden unter schnellem Rühren auf 155°C erhitzt. Die Reaktion ist nach 8 Stunden (GC-Kontrolle) abgeschlossen. Durch Destillation im Vakuum (1 mbar) bei 100°C über eine Kolonne direkt aus dem Reaktionsgemisch, erhält man

28 g des Produktes, das noch ca 20-30 % Sulpholan enthält. Nach der zweiten Destillation über eine Vigreux Kolonne erhält man : 15,68 g des Produktes (80 % Ausbeute) in Form eines Öls.
GC/MS : m/z 196.

## Patentansprüche

1. Verfahren zum Herstellen von 5-Fluor-1-alkyl-3-fluoralkyl-1H-pyrazol-4-carbonsäurehalogeniden der Formel (I), 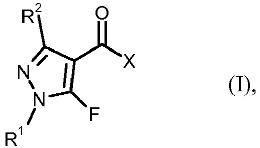 in welcher R¹ für C₁-C₆-Alkyl, R² für C₁-C₅-Fluoralkyl und X für Fluor oder Chlor steht, umfassend die Schritte
(1) Chlorierung von Alkyl 1-alkyl-3-fluoralkyl-1H-pyrazol-4-carboxylaten der Formel (II), 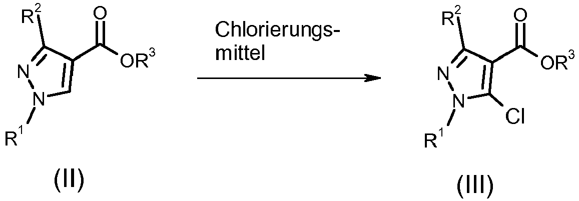 in der R¹, R² die oben angegebenen Bedeutungen haben und R³ eine lineare oder verzweigte C₁₋₁₂-Alkylgruppe ist, in Gegenwart eines Chlorierungsmittels zu Alkyl 5-Chlor-1-alkyl-3-fluoralkyl-1H-pyrazol-4-carboxylaten der Formel (III), in der R¹, R² und R³ die oben angegebenen Bedeutungen haben;
(2a-i) Fluorierung der Alkyl 5-Chlor-1-alkyl-3-fluoralkyl-1H-pyrazol-4-carboxylate der Formel (III) in Gegenwart eines Fluorierungsmittels der Formel (IV), wobei M⁺ für Li⁺, Na⁺, K⁺, Cs⁺, Alk₄N⁺ steht und Alk für C₁-C₄-Alkyl steht, zu Alkyl 5-Fluor-1-alkyl-3-fluoralkyl-1H-pyrazol-4-carboxylaten der Formel (V) und
(2a-ii) Hydrolyse der Alkyl 5-Fluor-1-alkyl-3-fluoralkyl-1H-pyrazol-4-carboxylate der Formel (V) zu 5-Fluor-1-alkyl-3-fluoralkyl-1H-pyrazol-4-carbonsäuren der Formel (VIa) und
(2a-iii) anschließende Halogenierung zu 5-Fluor-1-alkyl-3-fluoralkyl-1H-pyrazol-4-carbonsäurehalogeniden der Formel (I), wobei X für Fluor oder Chlor steht; 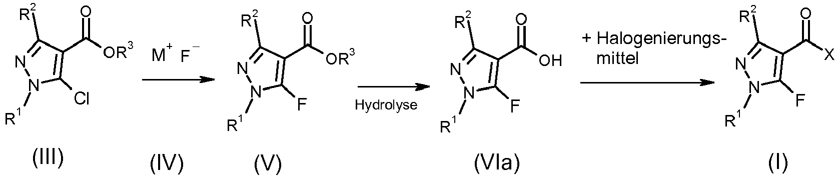 oder
(2b-i) Hydrolyse der Alkyl 5-Chlor-1-alkyl-3-fluoralkyl-1H-pyrazol-4-carboxylate der Formel (III) zu 5-Chlor-1-alkyl-3-fluoralkyl-1H-pyrazol-4-carbonsäuren der Formel (VI),
(2b-ii) anschließende Halogenierung der 5-Chlor-1-alkyl-3-fluoralkyl-1H-pyrazol-4-carbonsäuren der Formel (VIb) zu 5-Chlor-1-alkyl-3-fluoralkyl-1H-pyrazol-4-carbonsäurehalogeniden der Formel (VII) und 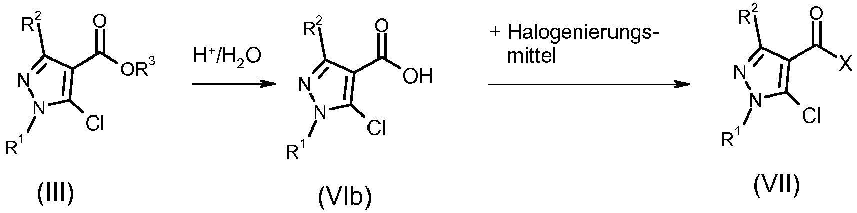
(2b-iii) Fluorierung der 5-Chlor-1-alkyl-3-fluoralkyl-1H-pyrazol-4-carbonsäurehalogeniden der Formel (VII) in Gegenwart eines Fluorierungsmittels der Formel (IV), wobei M⁺ für Li⁺, Na⁺, K⁺, Cs⁺, Alk₄N⁺ steht und Alk für C₁-C₄-Alkyl steht, zu den 5-Fluor-1-alkyl-3-fluoralkyl-1H-pyrazol-4-carbonsäurefluoriden der Formel (I), wobei X für Fluor steht.

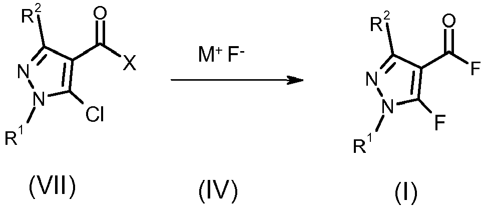

2. Verfahren gemäß Anspruch 1, **dadurch gekennzeichnet, dass** der Fluorierungsschritt (2a) oder (2b-ii) in Gegenwart eines Phasen-Transfer-Katalysators erfolgt.

3. Verfahren gemäß Anspruch 2, **dadurch gekennzeichnet, dass** der Phasen-Transfer-Katalysator aus quarternären Ammonium-, Phosphoniumverbindungen oder Amidophosphonium-Salzen ausgewählt ist.

4. Verfahren gemäß Anspruch 2 oder 3, **dadurch gekennzeichnet, dass** der Phasen-Transfer-Katalysator ausgewählt ist aus Tetramethylammonimchlorid oder Bromid, Tetrabutylammoniumchlorid, Trimethylbenzylammoniumchlorid, Tetrabutylammoniumbromid, Tetrabutylphosphoniumchlorid, Tetrabutylphosphoniumbromid, Tetraphenylphosphoniumchlorid oder Tetraphenylphosphoniumbromid, Tetrakis(dimethylamino)phosphoniumchlorid oder - bromid, Tetrakis(diethylamino)phosphoniumchlorid oder -bromid, Tris(diethylamino)-(dimethylamino)phosphoniumchlorid oder -bromid, Tris(dimethylamino)(dihexylamino)-phosphoniumchlorid oder -bromid, Tris(diethylamino)(dihexylamino)phosphoniumchlorid oder -bromid, Hexaalkyguanidiniusalze oder Polyethylenglykoldimethylethern der Kettenlängen r von 6 bis 17 und einer mittleren Molmasse von 500 g/mol, genannt.

5. Verfahren gemäß einem der Ansprüche 1 bis 4, **dadurch gekennzeichnet, dass** das Alkyl 5-Chlor-1-alkyl-3-fluoralkyl-1H-pyrazol-4-carboxylat der Formel (III) Ethyl 1-Methyl-3-difluormethyl-1H-pyrazol-4-carboxylat ist.

6. Ethyl 5-Chlor-1-methyl-3-difluormethyl-1H-pyrazol-4-carboxylat.

7. Ethyl 5-Fluor-1-methyl-3-difluormethyl-1H-pyrazol-4-carboxylat.

## Claims

1. Process for the preparation of 5-fluoro-1-alkyl-3-fluoroalkyl-1H-pyrazole-4-carbonyl halides of the formula (I) 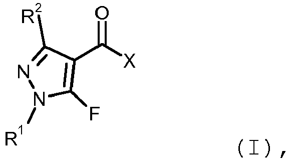 in which R¹ is C₁-C₆-alkyl, R² is C₁-C₅-fluoroalkyl and X is fluorine or chlorine, comprising the stages
(1)chlorination of alkyl 1-alkyl-3-fluoroalkyl-1H-pyrazole-4-carboxylates of the formula (II) 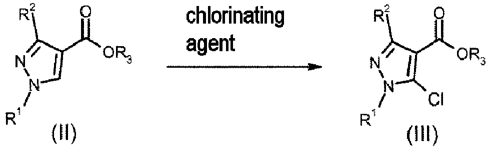 in which R¹ and R² have the meanings given above
and R³ is a linear or branched C₁₋₁₂-alkyl group, in the presence of a chlorinating agent to give alkyl 5-chloro-1-alkyl-3-fluoroalkyl-1H-pyrazole-4-carboxylates of the formula (III), in which R¹, R² and R³ have the meanings given above;
(2a-i) fluorination of the alkyl 5-chloro-1-alkyl-3-fluoroalkyl-1H-pyrazole-4-carboxylates of the formula (III) in the presence of a fluorinating agent of the formula (IV), in which M⁺ is Li⁺, Na⁺, K⁺, Cs⁺ or Alk₄N⁺ and Alk is C₁-C₄-alkyl, to give alkyl 5-fluoro-1-alkyl-3-fluoroalkyl-1H-pyrazole-4-carboxylates of the formula (V) and
(2a-ii) hydrolysis of the alkyl 5-fluoro-1-alkyl-3-fluoroalkyl-1H-pyrazole-4-carboxylates of the formula (V) to give 5-fluoro-1-alkyl-3-fluoroalkyl-1H-pyrazole-4-carboxylic acids of the formula (VIa) and
(2a-iii) subsequent halogenation to give 5-fluoro-1-alkyl-3-fluoroalkyl-1H-pyrazole-4-carbonyl halides of the formula (I), in which X is fluorine or chlorine; 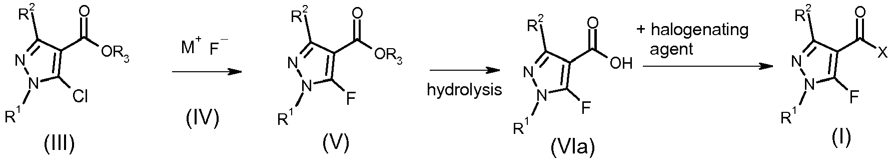 or
(2b-i) hydrolysis of the alkyl 5-chloro-1-alkyl-3-fluoroalkyl-1H-pyrazole-4-carboxylates of the formula (III) to give 5-chloro-1-alkyl-3-fluoroalkyl-1H-pyrazole-4-carboxylic acids of the formula (VI),
(2b-ii) subsequent halogenation of the 5-chloro-1-alkyl-3-fluoroalkyl-1H-pyrazole-4-carboxylic acids of the formula (VIb) to give 5-chloro-1-alkyl-3-fluoroalkyl-1H-pyrazole-4-carbonyl halides of the formula (VII) and 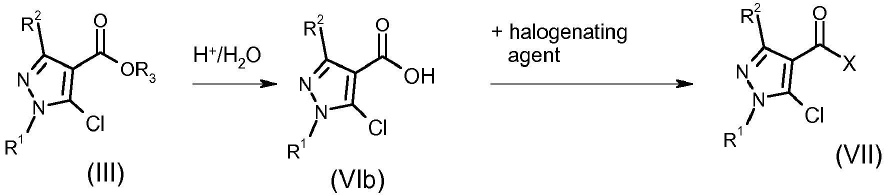
(2b-iii) fluorination of the 5-chloro-1-alkyl-3-fluoroalkyl-1H-pyrazole-4-carbonyl halides of the formula (VII) in the presence of a fluorinating agent of the formula (IV), in which M⁺ is Li⁺, Na⁺, K⁺, Cs⁺ or Alk₄N⁺ and Alk is C₁-C₄-alkyl, to give the 5-fluoro-1-alkyl-3-fluoroalkyl-1H-pyrazole-4-carbonyl fluorides of the formula (I), in which X is fluorine.

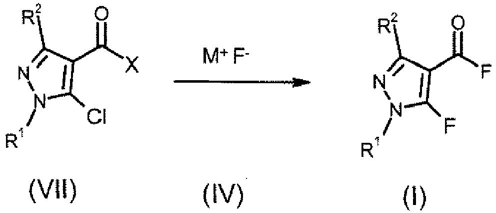

2. Process according to Claim 1, **characterized in that** the fluorination stage (2a) or (2b-ii) is carried out in the presence of a phase transfer catalyst.

3. Process according to Claim 2, **characterized in that** the phase transfer catalyst is chosen from quaternary ammonium or phosphonium compounds or amidophosphonium salts.

4. Process according to Claim 2 or 3, **characterized in that** the phase transfer catalyst is chosen from tetramethylammonium chloride or bromide, tetrabutylammonium chloride, trimethylbenzylammonium chloride, tetrabutylammonium bromide, tetrabutylphosphonium chloride, tetrabutylphosphonium bromide, tetraphenylphosphonium chloride, tetraphenylphosphonium bromide, tetrakis(dimethylamino)phosphonium chloride or bromide, tetrakis(diethylamino)phosphonium chloride or bromide, tris(diethyl-amino)(dimethylamino)phosphonium chloride or bromide, tris(dimethylamino)(dihexyl-amino)phosphonium chloride or bromide, tris(diethylamino)(dihexylamino)phosphonium chloride or bromide, hexaalkylguanidinium salts or polyethylene glycol dimethyl ethers with chain lengths r of 6 to 17 and an average molar mass of 500 g/mol.

5. Process according to one of Claims 1 to 4, **characterized in that** the alkyl 5-chloro-1-alkyl-3-fluoroalkyl-1H-pyrazol-4-carboxylate of the formula (III) is ethyl 1-methyl-3-difluoromethyl-1H-pyrazole-4-carboxylate.

6. Ethyl 5-chloro-1-methyl-3-difluoromethyl-1H-pyrazole-4-carboxylate.

7. Ethyl 5-fluoro-1-methyl-3-difluoromethyl-1H-pyrazole-4-carboxylate.

## Revendications

1. Procédé pour la préparation d'halogénures de l'acide 5-fluoro-1-alkyl-3-fluoroalkyl-1H-pyrazole-4-carboxylique de formule (I), 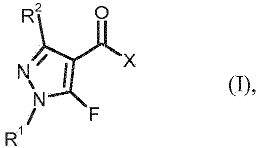 dans laquelle R¹ représente C₁-C₆-alkyle, R² représente C₁-C₅-fluoroalkyle et X représente fluor ou chlore, comprenant les étapes de
(1) chloration de 1-alkyl-3-fluoroalkyl-1H-pyrazole-4-carboxylates d'alkyle de formule (II), 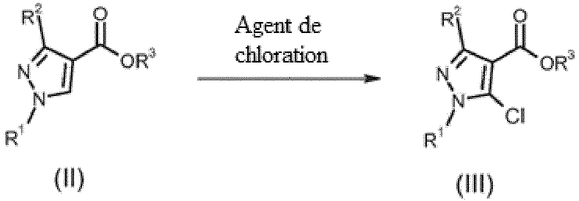 dans laquelle R¹, R² présentent les significations indiquées ci-dessus et R³ représente un groupe C₁₋₁₂-alkyle linéaire ou ramifié, en présence d'un agent de chloration en 5-chloro-1-alkyl-3-fluoroalkyl-1H-pyrazole-4-carboxylates d'alkyle de formule (III), dans laquelle R¹, R² et R³ présentent les significations indiquées ci-dessus ;
(2a-i) fluoration des 5-chloro-1-alkyl-3-fluoroalkyl-1H-pyrazole-4-carboxylates d'alkyle de formule (III) en présence d'un agent de fluoration de formule (IV), M⁺ représentant Li⁺, Na⁺, K⁺, Cs⁺, Alk₄N⁺ et Alk représentant C₁-C₄-alkyle, en 5-fluoro-1-alkyl-3-fluoroalkyl-1H-pyrazole-4-carboxylates d'alkyle de formule (V) et
(2a-ii) hydrolyse des 5-fluoro-1-alkyl-3-fluoroalkyl-1H-pyrazole-4-carboxylates d'alkyle de formule (V) en acides 5-fluoro-1-alkyl-3-fluoroalkyl-1H-pyrazole-4-carboxyliques de formule (VIa) et
(2a-iii) halogénation consécutive en halogénures de l'acide 5-fluoro-1-alkyl-3-fluoroalkyl-1H-pyrazole-4-carboxylique de formule (I), X représentant fluor ou chlore ; 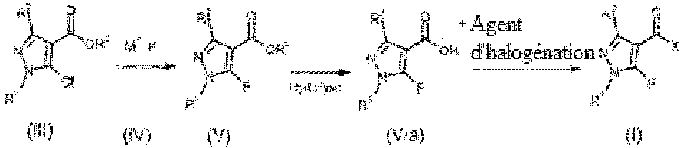 ou
(2b-i) hydrolyse des 5-chloro-1-alkyl-3-fluoroalkyl-1H-pyrazole-4-carboxylates d'alkyle de formule (III) en acides 5-chloro-1-alkyl-3-fluoroalkyl-1H-pyrazole-4-carboxyliques de formule (VI),
(2b-ii) halogénation consécutive des acides 5-chloro-1-alkyl-3-fluoroalkyl-1H-pyrazole-4-carboxyliques de formule (VIb) en halogénures de l'acide 5-chloro-1-alkyl-3-fluoroalkyl-1H-pyrazole-4-carboxylique de formule (VII) et 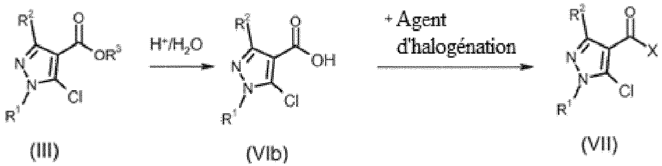
(2b-iii) fluoration des halogénures de l'acide 5-chloro-1-alkyl-3-fluoroalkyl-1H-pyrazole-4-carboxylique de formule (VII) en présence d'un agent de fluoration de formule (IV), M⁺ représentant Li⁺, Na⁺, K⁺, Cs⁺, Alk₄N⁺ et Alk représentant C₁-C₄-alkyle, en fluorures de l'acide 5-fluoro-1-alkyl-3-fluoroalkyl-1H-pyrazole-4-carboxylique de formule (I), X représentant fluor.

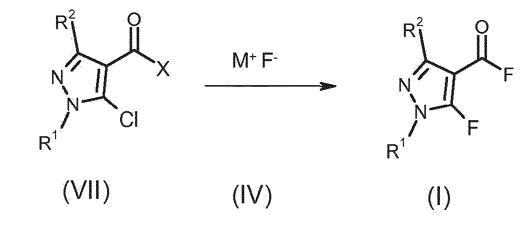

2. Procédé selon la revendication 1, **caractérisé en ce que** l'étape de fluoration (2a) ou (2b-ii) a lieu en présence d'un catalyseur de transfert de phase.

3. Procédé selon la revendication 2, **caractérisé en ce que** le catalyseur de transfert de phase est choisi parmi les composés d'ammonium quaternaire, de phosphonium quaternaire ou les sels d'amidophosphonium.

4. Procédé selon la revendication 2 ou 3, **caractérisé en ce que** le catalyseur de transfert de phase est choisi parmi le chlorure ou le bromure de tétraméthylammonium, le chlorure de tétrabutylammonium, le chlorure de triméthylbenzylammonium, le bromure de tétrabutylammonium, le chlorure de tétrabutylphosphonium, le bromure de tétrabutylphosphonium, le chlorure de tétraphénylphosphonium ou le bromure de tétraphénylphosphonium, le chlorure ou le bromure de tétrakis(diméthylamino)phosphonium, le chlorure ou le bromure de tétrakis(diéthylamino)phosphonium, le chlorure ou le bromure de tris(diéthylamino)-(diméthylamino)phosphonium, le chlorure ou le bromure de tris(diméthylamino)(dihexylamino)phosphonium, le chlorure ou le bromure de tris(diéthylamino)(dihexylamino)phosphonium, les sels d'hexaalkylguanidinium ou les polyéthylèneglycoldiméthyléthers présentant des longueurs de chaîne r de 6 à 17 et une masse molaire moyenne de 500 g/mole.

5. Procédé selon l'une quelconque des revendications 1 à 4, **caractérisé en ce que** le 5-chloro-1-alkyl-3-fluoroalkyl-1H-pyrazole-4-carboxylate d'alkyle de formule (III) est le 1-méthyl-3-difluorométhyl-1H-pyrazole-4-carboxylate d'éthyle.

6. 5-chloro-1-méthyl-3-difluorométhyl-1H-pyrazole-4-carboxylate d'éthyle.

7. 5-fluoro-1-méthyl-3-difluorométhyl-1H-pyrazole-4-carboxylate d'éthyle.
